# EUROPEAN PATENT SPECIFICATION

(11) **EP 3 699 564 B1**
(45) Date of publication and mention of the grant of the patent: **06.04.2022**
(21) Application number: 19159133.8
(22) Date of filing: 25.02.2019
(51) Int. Cl.: G01J 5/02, G01J 5/20, G01J 5/00, G01J 5/06, A61B 5/01, G01K 13/00

(54) **INFRARED THERMOMETER CAPABLE OF SWITCHING FOREHEAD TEMPERATURE MEASUREMENT MODE AND EAR TEMPERATURE MEASUREMENT MODE AND SWITCHING METHOD**
INFRAROTTHERMOMETER MIT FÄHIGKEIT ZUR UMSCHALTUNG DES STIRNTEMPERATURMESSMODUS UND OHRTEMPERATURMESSMODUS UND UMSCHALTVERFAHREN
THERMOMÈTRE À INFRAROUGES CAPABLE DE COMMUTER LE MODE DE MESURE DE LA TEMPÉRATURE DU FRONT ET LE MODE DE MESURE DE LA TEMPÉRATURE DE L'OREILLE ET SON PROCÉDÉ DE COMMUTATION

(43) Date of publication of application: 26.08.2020
(73) Proprietor: Hetaida Technology Co., Ltd., 523000 Dongguan City, Guangdong (CN)
(72) Inventor: CHEN, Zhenguang, Dongguan City, Guangdong 523000 (CN)
(74) Representative: Cabinet Chaillot

(56) References cited:
- CN-U- 203 745 084
- CN-U- 207 866 369
- US-A1- 2004 047 392

## Description

### BACKGROUND OF THE INVENTION

### 1. Field of the Invention

The present invention relates to a device for measuring body temperature, and more particularly to an infrared thermometer capable of switching a forehead temperature measurement mode and an ear temperature measurement mode and a switching method.

### 2. Description of the Prior Art

An infrared thermometer that can switch a forehead temperature measurement mode and an ear temperature measurement mode combines an infrared ear thermometer and an infrared forehead thermometer. In the prior art, there are two ways for switching the forehead temperature measurement mode and the ear temperature measurement mode. One is provided with a switch. The forehead temperature measurement mode and the ear temperature measurement mode are switched manually through the switch. The other is to determine whether there is a cover by identification. When the infrared thermometer is connected with the cover, the forehead temperature measurement mode is performed. When the infrared thermometer is not connected with the cover, the ear temperature measurement mode is performed. There are some deficiencies in the above ways. For example, the switching operation is troublesome. The way to determine the existence of the cover relies on a relatively complicated structural design for a mechanical connection, which is troublesome in production. It is prone to have problems, such as unreliable mechanical contact.

In addition, the forehead temperature measurement is affected by the ambient environment greatly. Clinically, there is an error in the measured forehead temperature value.

CN 203 745 084 U discloses an infrared temperature measuring device according to the preamble of claim 1, and it only discloses that the forehead/ear temperature measurement modes can be switched by removing or installing the forehead probe cover.

Accordingly, the inventor of the present invention has devoted himself based on his many years of practical experiences to solve these problems.

### SUMMARY OF THE INVENTION

In view of the shortcomings of the prior art, the primary object of the present invention is to provide an infrared thermometer capable of switching a forehead temperature measurement mode and an ear temperature measurement mode and a switching method. The switching control is simple and convenient, and the accuracy of the forehead temperature measurement is improved effectively.

In order to achieve the above object, the present invention adopts the following technical solutions:
According to one aspect of the present invention, an infrared thermometer capable of switching a forehead temperature measurement mode and an ear temperature measurement mode is provided. The infrared thermometer comprises an infrared thermometer body and a forehead temperature measurement cover. One end of the infrared thermometer body has a measuring probe. The infrared thermometer body includes a printed circuit board therein. The measuring probe is connected to the printed circuit board. The forehead temperature measurement cover is detachably connected to the infrared thermometer body to selectively cover or expose the measuring probe for switching the forehead temperature measurement mode and the ear temperature measurement mode. The printed circuit board is connected with a first electrical contact. The infrared thermometer is characterized in that the forehead temperature measurement cover has a thermistor and a second electrical contact connected to the thermistor.. When the forehead temperature measurement cover is connected to the infrared thermometer body, the second electrical contact and the first electrical contact form an electrical connection so that the thermistor is connected to the printed circuit board, and the thermometer switches to a forehead temperature measurement mode once the thermistor is connected to the printed circuit board.

Preferably, one of the infrared thermometer body and the forehead temperature measurement cover has an engaging protrusion, and the other has an engaging recess. Through the engaging protrusion fitted to the engaging recess, the forehead temperature measurement cover and the infrared thermometer body are connected together and positioned.

Preferably, the first electrical contact and the second electrical contact are disposed on the engaging protrusion and the engaging recess, respectively. The electrical connection between the first electrical contact and the second electrical contact is realized while the infrared thermometer body and the forehead temperature measurement cover are positioned mechanically.

Preferably, the infrared thermometer body is provided with an annular raised platform corresponding to a rear end of the measuring probe. The infrared thermometer body has an annular groove around an outer circumference of the annular raised platform. The engaging protrusion is exposed in the annular groove. The engaging recess is formed on an inner wall of the forehead temperature measurement cover. The forehead temperature measurement cover is fitted to the annular groove.

According to another aspect of the present invention, a method for switching between a forehead temperature measurement mode and an ear temperature measurement mode in the aforesaid infrared thermometer is provided. When the forehead temperature measurement cover is connected to the infrared thermometer body, the second electrical contact and the first electrical contact form the electrical connection so that the thermistor is connected to the printed circuit board, and the printed circuit board determines that the forehead temperature measurement mode is performed to obtain a forehead temperature value by determining that the thermistor is connected to the printed circuit board; and the printed circuit board acquires a resistance value of the thermistor to calculate and obtain an ambient temperature and compensates the forehead temperature value measured by the infrared thermometer body by using the ambient temperature to obtain a calibrated forehead temperature value. When the forehead temperature measurement cover is not connected to the infrared thermometer body, the measuring probe is exposed, the printed circuit board is not connected with the thermistor, and the printed circuit board determines that the ear temperature measurement mode is performed.

Preferably, the forehead temperature measurement cover is connected to the infrared thermometer body in a pluggable manner. When the forehead temperature measurement cover and the infrared thermometer body are connected mechanically, the first electrical contact and the second electrical contact form the electrical connection.

Compared with the prior art, the present invention has obvious advantages and beneficial effects. Specifically, it can be known from the above technical solutions. The forehead temperature measurement cover is provided with the thermistor. The thermistor is used as the detecting component for switching the forehead temperature measurement mode and the ear temperature measurement mode. Besides, the thermistor is used as the component for detecting the ambient temperature. The ambient temperature is fed back to compensate the forehead temperature value measured by the infrared thermometer body, thereby obtaining the calibrated forehead temperature value and improving the accuracy of the forehead temperature measurement.

Embodiments of the present invention will now be described, by way of example only, with reference to the accompanying drawings.

### BRIEF DESCRIPTION OF THE DRAWINGS

FIG. 1 is an exploded view in accordance with an embodiment of the present invention;
FIG. 2 is another exploded view in accordance with an embodiment of the present invention; and
FIG. 3 is a block diagram showing a circuit connection in accordance with an embodiment of the present invention.

### DETAILED DESCRIPTION OF THE PREFERRED EMBODIMENTS

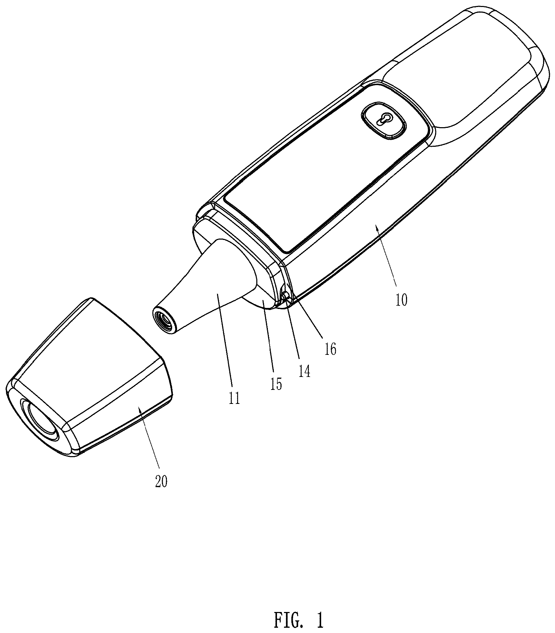

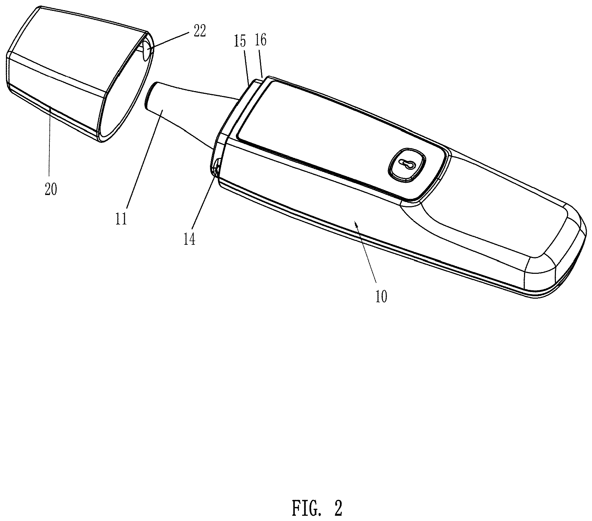

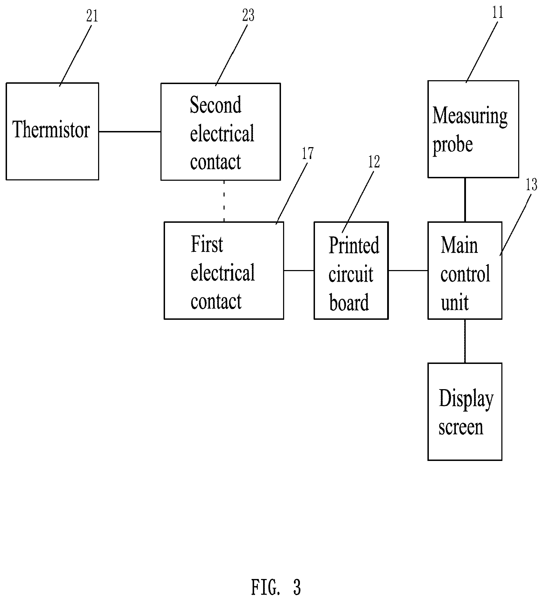

Referring to FIG. 1 to FIG. 3, there is shown a specific structure of an embodiment of the present invention.

An infrared thermometer capable of switching a forehead temperature measurement mode and an ear temperature measurement mode comprises an infrared thermometer body 10 and a forehead temperature measurement cover 20. The infrared thermometer body 10 is provided with a power switch, a display screen, and the like.

One end of the infrared thermometer body 10 has a measuring probe 11. The infrared thermometer body 10 includes a printed circuit board 12 therein. The measuring probe 11 is connected to the printed circuit board 12. The printed circuit board 12 is connected with a first electrical contact 17.

The forehead temperature measurement cover 20 has a thermistor 21 and a second electrical contact 23 connected to the thermistor 21.

The forehead temperature measurement cover 20 is detachably connected to the infrared thermometer body 10 to selectively cover or expose the measuring probe 11 for switching the forehead temperature measurement mode and the ear temperature measurement mode. When the forehead temperature measurement cover 20 is connected to the infrared thermometer body 10, the second electrical contact 23 and the first electrical contact 17 form an electrical connection so that the thermistor 21 is connected to the printed circuit board 12.

When the forehead temperature measurement cover 20 is connected to the infrared thermometer body 10, the second electrical contact 23 and the first electrical contact 17 form the electrical connection so that the thermistor 21 is connected to the printed circuit board 12, and the printed circuit board 12 determines that the forehead temperature measurement mode is performed to obtain a forehead temperature value; and the printed circuit board 12 acquires the resistance value of the thermistor 21 to calculate and obtain the ambient temperature and compensates the forehead temperature value measured by the infrared thermometer body 10 by using the ambient temperature to obtain a calibrated forehead temperature value.

When the forehead temperature measurement cover 20 is not connected to the infrared thermometer body 10, the measuring probe 11 is exposed, the printed circuit board 12 is not connected with the thermistor 21, and the printed circuit board 12 determines that the ear temperature measurement mode is performed.

Therefore, in this embodiment, the forehead temperature measurement mode or the ear temperature measurement mode is determined by whether the thermistor 21 is connected to the printed circuit board 12. The printed circuit board 12 is connected with a main control unit 13 for switching the corresponding forehead temperature measurement mode and the ear temperature measurement mode automatically. There is no need to control the switch manually, without relying on a complex mechanical structure. The present invention improves the convenience and reliability of switching effectively.

Generally, the forehead temperature measurement cover 20 is connected to the infrared thermometer body 10 in a pluggable manner. The mechanical connection of the forehead temperature measurement cover 20 and the infrared thermometer body 10 and the electrical connection of the first and second electrical contacts 17, 23 are completed by one insertion operation.

The pluggable structure between the forehead temperature measurement cover 20 and the infrared thermometer body 10 may be implemented in different manners. In this embodiment, a feasible structure is provided for explanation. One of the infrared thermometer body 10 and the forehead temperature measurement cover 20 has an engaging protrusion 14, and the other has an engaging recess 22. Through the engaging protrusion 14 fitted to the engaging recess 22, the forehead temperature measurement cover 20 and the infrared thermometer body 10 are connected together and positioned. The first electrical contact 17 and the second electrical contact 23 are disposed on the engaging protrusion 14 and the engaging recess 22, respectively. The electrical connection between the first electrical contact 17 and the second electrical contact 23 is realized while the infrared thermometer body 10 and the forehead temperature measurement cover 20 are positioned mechanically. The first electrical contact and the second electrical contact may be disposed at other positions, not limited to the positions of the engaging protrusion 14 and the engaging recess 22. The electrical contacts may be a resilient boss to provide better and reliable contact between the first electrical contact and the second electrical contact.

Furthermore, the infrared thermometer body 10 is provided with an annular raised platform 15 corresponding to the rear end of the measuring probe 11. The infrared thermometer body 10 has an annular groove 16 around the outer circumference of the annular raised platform 15. The engaging protrusion 14 is exposed in the annular groove 16. The engaging recess 22 is formed on the inner wall of the forehead temperature measurement cover 20. The forehead temperature measurement cover 20 is fitted to the annular groove 16.

The feature of the present invention is described below. The forehead temperature measurement cover is provided with the thermistor. The thermistor is used as the detecting component for switching the forehead temperature measurement mode and the ear temperature measurement mode. Besides, the thermistor is used as the component for detecting the ambient temperature. The ambient temperature is fed back to compensate the forehead temperature value measured by the infrared thermometer body, thereby obtaining the calibrated forehead temperature value and improving the accuracy of the forehead temperature measurement.

Although particular embodiments of the present invention have been described in detail for purposes of illustration, various modifications and enhancements may be made as long as they fall within the scope of the appended claims. without Accordingly, the present invention is not to be limited except as by the appended claims.

## Claims

1. An infrared thermometer capable of switching a forehead temperature measurement mode and an ear temperature measurement mode, comprising an infrared thermometer body (10) and a forehead temperature measurement cover (20); wherein
one end of the infrared thermometer body (10) has a measuring probe (11), the infrared thermometer body (10) includes a printed circuit board (12) therein, the measuring probe (11) is connected to the printed circuit board (12), the forehead temperature measurement cover (20) is detachably connected to the infrared thermometer body (10) to selectively cover or expose the measuring probe (11) for switching the forehead temperature measurement mode and the ear temperature measurement mode;
the printed circuit board (12) is connected with a first electrical contact (17);
**characterized in that**:
the forehead temperature measurement cover (20) has a thermistor (21) and a second electrical contact (23) connected to the thermistor (21); and
when the forehead temperature measurement cover (20) is connected to the infrared thermometer body (10), the second electrical contact (23) and the first electrical contact (17) form an electrical connection so that the thermistor (21) is connected to the printed circuit board (12), and the thermometer switches to a forehead temperature measurement mode once the thermistor (21) is connected to the printed circuit board (12).

2. The infrared thermometer as claimed in claim 1, wherein one of the infrared thermometer body (10) and the forehead temperature measurement cover (20) has an engaging protrusion (14), and the other has an engaging recess (22); through the engaging protrusion (14) fitted to the engaging recess (22), the forehead temperature measurement cover (20) and the infrared thermometer body (10) are connected together and positioned.

3. The infrared thermometer as claimed in claim 2, wherein the first electrical contact (17) and the second electrical contact (23) are disposed on the engaging protrusion (14) and the engaging recess (22) respectively, the electrical connection between the first electrical contact (17) and the second electrical contact (23) is realized while the infrared thermometer body (10) and the forehead temperature measurement cover (20) are positioned mechanically.

4. The infrared thermometer as claimed in claim 3, wherein the infrared thermometer body (10) is provided with an annular raised platform (15) corresponding to a rear end of the measuring probe (11), the infrared thermometer body (10) has an annular groove (16) around an outer circumference of the annular raised platform (15); the engaging protrusion (14) is exposed in the annular groove (16), the engaging recess (22) is formed on an inner wall of the forehead temperature measurement cover (20), and the forehead temperature measurement cover (20) is fitted to the annular groove (16).

5. A method for switching between a forehead temperature measurement mode and an ear temperature measurement mode in the infrared thermometer as claimed in claim 1, **characterized in that**:
when the forehead temperature measurement cover (20) is connected to the infrared thermometer body (10), the second electrical contact (23) and the first electrical contact (17) form the electrical connection so that the thermistor (21) is connected to the printed circuit board (12), and the printed circuit board (12) determines that the forehead temperature measurement mode is performed to obtain a forehead temperature value by determining that the thermistor (21) is connected to the printed circuit board (12); and the printed circuit board (12) acquires a resistance value of the thermistor (21) to calculate and obtain an ambient temperature and compensates the forehead temperature value measured by the infrared thermometer body (10) by using the ambient temperature to obtain a calibrated forehead temperature value;
when the forehead temperature measurement cover (20) is not connected to the infrared thermometer body (10), the measuring probe (11) is exposed, the printed circuit board (12) is not connected with the thermistor (21), and the printed circuit board (12) determines that the ear temperature measurement mode is performed.

6. The method as claimed in claim 5, wherein the forehead temperature measurement cover (20) is connected to the infrared thermometer body (10) in a pluggable manner, when the forehead temperature measurement cover (20) and the infrared thermometer body (10) are connected mechanically, the first electrical contact (17) and the second electrical contact (23) form the electrical connection.

## Patentansprüche

1. Infrarotthermometer, das in der Lage ist, zwischen einem Stirntemperatur-Messmodus und einem Ohrtemperatur-Messmodus umzuschalten, einen Infrarotthermometer-Hauptteil (10) und eine Abdeckung (20) für die Stirntemperaturmessung umfassend, wobei:
ein Ende des Infrarotthermometer-Hauptteils (10) eine Messsonde (11) aufweist, der Infrarotthermometer-Hauptteil (10) eine Leiterplatte (12) darin beinhaltet, die Messsonde (11) mit der Leiterplatte (12) verbunden ist, die Abdeckung (20) für die Stirntemperaturmessung lösbar mit dem Infrarotthermometer-Hauptteil (10) verbunden ist, um die Messsonde (11) wahlweise abzudecken oder freizulegen, um zwischen dem Stirntemperatur-Messmodus und dem Ohrtemperatur-Messmodus umzuschalten,
die Leiterplatte (12) mit einem ersten elektrischen Kontakt (17) verbunden ist,
**dadurch gekennzeichnet, dass**:
die Abdeckung (20) für die Stirntemperaturmessung einen Thermistor (21) und einem zweiten elektrischen Kontakt (23) aufweist, welcher mit dem Thermistor (21) verbunden ist, und
der zweite elektrische Kontakt (23) und der erste elektrische Kontakt (17) eine elektrische Verbindung bilden, wenn die Abdeckung (20) für die Stirntemperaturmessung mit dem Infrarotthermometer-Hauptteil (10) verbunden ist, so dass der Thermistor (21) mit der Leiterplatte (12) verbunden ist und das Thermometer in einen Stirntemperatur-Messmodus schaltet, sobald der Thermistor (21) mit der Leiterplatte (12) verbunden ist.

2. Infrarotthermometer nach Anspruch 1, wobei eines von dem Infrarotthermometer-Hauptteil (10) und der Abdeckung (20) für die Stirntemperaturmessung einen Eingriffsvorsprung (14) aufweist und das andere eine Eingriffsvertiefung (22) aufweist, wobei durch den in die Eingriffsvertiefung (22) eingesetzten Eingriffsvorsprung (14) die Abdeckung (20) für die Stirntemperaturmessung und der Infrarotthermometer-Hauptteil (10) miteinander verbunden und positioniert sind.

3. Infrarotthermometer nach Anspruch 2, wobei der erste elektrische Kontakt (17) und der zweite elektrische Kontakt (23) an dem Eingriffsvorsprung (14) beziehungsweise der Eingriffsvertiefung (22) angeordnet sind, wobei die elektrische Verbindung zwischen dem ersten elektrischen Kontakt (17) und dem zweiten elektrischen Kontakt (23) realisiert ist, während der Infrarotthermometer-Hauptteil (10) und die Abdeckung (20) für die Stirntemperaturmessung mechanisch positioniert worden sind.

4. Infrarotthermometer nach Anspruch 3, wobei der Infrarotthermometer-Hauptteil (10) mit einem ringförmigen erhöhten Absatz (15) versehen ist, der einem hinteren Ende der Messsonde (11) entspricht, wobei der Infrarotthermometer-Hauptteil (10) rings um einen Außenumfang des ringförmigen erhöhten Absatzes (15) eine ringförmige Auskehlung (16) aufweist, wobei der Eingriffsvorsprung (14) in der ringförmigen Auskehlung (16) freiliegt, die Eingriffsvertiefung (22) an einer inneren Wandung der Abdeckung (20) für die Stirntemperaturmessung gebildet ist und die Abdeckung (20) für die Stirntemperaturmessung auf die ringförmige Auskehlung (16) aufgesetzt ist.

5. Verfahren zum Umschalten zwischen einem Stirntemperatur-Messmodus und einem Ohrtemperatur-Messmodus in dem Infrarotthermometer nach Anspruch 1, **dadurch gekennzeichnet, dass**:
der zweite elektrische Kontakt (23) und der erste elektrische Kontakt (17) die elektrische Verbindung bilden, wenn die Abdeckung (20) für die Stirntemperaturmessung mit dem Infrarotthermometer-Hauptteil (10) verbunden wird, so dass der Thermistor (21) mit der Leiterplatte (12) verbunden wird und die Leiterplatte (12) durch das Bestimmen, dass der Thermistor (21) mit der Leiterplatte (12) verbunden ist, bestimmt, dass der Stirntemperatur-Messmodus ausgeführt wird, um einen Stirntemperaturwert zu erzielen, und die Leiterplatte (12) einen Widerstandswert des Thermistors (21) erfasst, um eine Umgebungstemperatur zu berechnen und zu erzielen, und den Stirntemperaturwert, der durch den Infrarotthermometer-Hauptteil (10) gemessen wird, mit Hilfe der Umgebungstemperatur kompensiert, um einen kalibrierten Stirntemperaturwert zu erzielen,
die Leiterplatte (12) nicht mit dem Thermistor (21) verbunden ist, wenn die Abdeckung (20) für die Stirntemperaturmessung nicht mit dem Infrarotthermometer-Hauptteil (10) verbunden ist, die Messsonde (11) freiliegt, und die Leiterplatte (12) bestimmt, dass der Ohrtemperatur-Messmodus ausgeführt wird.

6. Verfahren nach Anspruch 5, wobei die Abdeckung (20) für die Stirntemperaturmessung mit dem Infrarotthermometer-Hauptteil (10) in einer steckbaren Weise verbunden wird, wenn die Abdeckung (20) für die Stirntemperaturmessung und der Infrarotthermometer-Hauptteil (10) mechanisch verbunden werden, wobei der erste elektrische Kontakt (17) und der zweite elektrische Kontakt (23) die elektrische Verbindung bilden.

## Revendications

1. Thermomètre infrarouge capable de passer entre un mode de mesure de température frontale et un mode de mesure de température auriculaire, comprenant un corps de thermomètre infrarouge (10) et un capuchon de mesure de température frontale (20) ; dans lequel
une extrémité du corps de thermomètre infrarouge (10) a une sonde de mesure (11), le corps de thermomètre infrarouge (10) comprend une carte de circuits imprimés (12) à l'intérieur de celui-ci, la sonde de mesure (11) est reliée à la carte de circuits imprimés (12), le capuchon de mesure de température frontale (20) est relié de manière détachable au corps de thermomètre infrarouge (10) pour recouvrir ou exposer de manière sélective la sonde de mesure (11) pour passer entre le mode de mesure de température frontale et le mode de mesure de température auriculaire ;
la carte de circuits imprimés (12) est reliée à un premier contact électrique (17) ;
**caractérisé par le fait que** :
le capuchon de mesure de température frontale (20) a une thermistance (21) et un second contact électrique (23) reliée à la thermistance (21) ; et
lorsque le capuchon de mesure de température frontale (20) est relié au corps de thermomètre infrarouge (10), le second contact électrique (23) et le premier contact électrique (17) forment une connexion électrique de telle sorte que la thermistance (21) est reliée à la carte de circuits imprimés (12), et le thermomètre passe à un mode de mesure de température frontale une fois que la thermistance (21) est reliée à la carte de circuits imprimés (12).

2. Thermomètre infrarouge selon la revendication 1, dans lequel l'un du corps de thermomètre infrarouge (10) et du capuchon de mesure de température frontale (20) a une saillie d'engagement (14), et l'autre a un évidement d'engagement (22) ; par l'intermédiaire de la saillie d'engagement (14) ajustée dans l'évidement d'engagement (22), le capuchon de mesure de température frontale (20) et le corps de thermomètre infrarouge (10) sont reliés ensemble et positionnés.

3. Thermomètre infrarouge selon la revendication 2, dans lequel le premier contact électrique (17) et le second contact électrique (23) sont disposés respectivement sur la saillie d'engagement (14) et l'évidement d'engagement (22), la connexion électrique entre le premier contact électrique (17) et le second contact électrique (23) est réalisée alors que le corps de thermomètre infrarouge (10) et le capuchon de mesure de température frontale (20) sont positionnés mécaniquement.

4. Thermomètre infrarouge selon la revendication 3, dans lequel le corps de thermomètre infrarouge (10) comporte une plate-forme surélevée annulaire (15) correspondant à une extrémité arrière de la sonde de mesure (11), le corps de thermomètre infrarouge (10) a une rainure annulaire (16) autour d'une circonférence externe de la plate-forme surélevée annulaire (15) ; la saillie d'engagement (14) est exposée dans la rainure annulaire (16), l'évidement d'engagement (22) est formé sur une paroi interne du capuchon de mesure de température frontale (20), et le capuchon de mesure de température frontale (20) est ajusté dans la rainure annulaire (16).

5. Procédé de commutation entre un mode de mesure de température frontale et un mode de mesure de température auriculaire dans le thermomètre infrarouge selon la revendication 1, **caractérisé par le fait que** :
lorsque le capuchon de mesure de température frontale (20) est relié au corps de thermomètre infrarouge (10), le second contact électrique (23) et le premier contact électrique (17) forment la connexion électrique de telle sorte que la thermistance (21) est reliée à la carte de circuits imprimés (12), et la carte de circuits imprimés (12) détermine que le mode de mesure de température frontale est exécuté pour obtenir une valeur de température frontale par détermination du fait que la thermistance (21) est reliée à la carte de circuits imprimés (12) ; et la carte de circuits imprimés (12) acquiert une valeur de résistance de la thermistance (21) pour calculer et obtenir une température ambiante et compense la valeur de température frontale mesurée par le corps de thermomètre infrarouge (10) en utilisant la température ambiante pour obtenir une valeur de température frontale calibrée ;
lorsque le capuchon de mesure de température frontale (20) n'est pas relié au corps de thermomètre infrarouge (10), la sonde de mesure (11) est exposée, la carte de circuits imprimés (12) n'est pas reliée à la thermistance (21), et la carte de circuits imprimés (12) détermine que le mode de mesure de température auriculaire est exécuté.

6. Procédé selon la revendication 5, dans lequel le capuchon de mesure de température frontale (20) est relié au corps de thermomètre infrarouge (10) de manière enfichable, lorsque le capuchon de mesure de température frontale (20) et le corps de thermomètre infrarouge (10) sont reliés mécaniquement le premier contact électrique (17) et le second contact électrique (23) forment la connexion électrique.
